Europäisches Patentamt

European Patent Office

Office européen des brevets

(19)

(11) Veröffentlichungsnummer : **0 084 547**
**B1**

(12) **EUROPÄISCHE PATENTSCHRIFT**

(45) Veröffentlichungstag der Patentschrift :
28.05.86

(21) Anmeldenummer : 82902351.4

(22) Anmeldetag : 06.08.82

(86) Internationale Anmeldenummer :
PCT/EP 82/00164

(87) Internationale Veröffentlichungsnummer :
WO/8300419 (17.02.83 Gazette 83/05)

(51) Int. Cl.⁴ : **A 23 L   1/20, A 01 N 65/00,
C 05 F 11/00, A 61 K 35/78**

(54) VERFAHREN UND VORRICHTUNG ZUM ENTZUG DER BITTERSTOFFE VON BITTERLUPINEN.

(30) Priorität : 06.08.81 DE 3131207
                19.01.82 DE 3201378
                21.05.82 DE 3219245

(43) Veröffentlichungstag der Anmeldung :
03.08.83 Patentblatt 83/31

(45) Bekanntmachung des Hinweises auf die Patenterteilung : 28.05.86 Patentblatt 86/22

(84) Benannte Vertragsstaaten :
AT BE CH DE FR GB LI LU NL SE

(56) Entgegenhaltungen :
WO-A-82 /021 49
DE-C-   329 201
DE-C-   350 100
DE-C-   531 828
Chemical Abstracts, vol. 43, 10 November 1949, no.
21, (Columbus, Ohio, US) G. Longo: "Action of the
aqueous extract of Lupinus albus on the movements
of the isolated uterus of the rabbit and guinea pig",
see column 8532c, Boll. soc. ital. biol. sper. 24, 1179-
81 (1948)
Chemical Abstracts, vol. 76, 13 March 1972, no. 11,
(Columbus, Ohio, US) B. Griffaut: "In vitro culture of
the cauline apical meristem of the red scarlet runner
(Phaseolus multiflorus) in the presence of a root
extract, gibberellin, and adenine", see page 239,
abstract 567237, C.R. Acad. Sci., Ser. D 1971, 273 (20)
1791-4 (Fr)

(73) Patentinhaber : MITTEX AKTIENGESELLSCHAFT
Aeulestrasse 5
FL-9490 Vaduz (LI)

(72) Erfinder : HUSSMANN, Peter
Via dei Pucci 4
I-50100 Florenz (IT)

(74) Vertreter : Haug, Dietmar et al
Patentanwälte Andrae, Flach, Haug Steinstrasse 44
D-8000 München 80 (DE)

## Beschreibung

Die Erfindung betrifft ein Verfahren zur Entbitterung der Samen von Bitterlupinen.

Die Bitterlupine ist eine Kulturpflanze, die schon von den alten Ägyptern und anschließend im gesamten Mittelmeerraum und in Südamerika angebaut wurde. Wegen ihres hohen, voll verdaulichen Eiweißgehalts von 40 % und mehr und eines Ölgehalts von 15 bis 20 % war und ist sie als Nahrungs- und Futtermittel geschätzt.

Der Einsatz der Bitterlupine als Nahrungs- und Futtermittel setzt allerdings voraus, daß ihr zunächst die Bitterstoffe entzogen werden, um nicht nur den äußerst bitteren Geschmack zu beseitigen, sondern um auch zu vermeiden, daß es durch die Bitterstoffe, insbesondere bei den Haustieren, zu der sog. Lupinenkrankheit kommen kann.

Ein herkömmliches Verfahren zur Entbitterung von Lupinen besteht darin, die Lupinen zunächst zu kochen und dann bis zu 48 Stunden zu wässern. Der Nachteil dieses Verfahrens besteht darin, daß es umständlich und kostspielig ist und außerdem einen erheblichen Nährwertverlust der Lupinen mit sich bringt. Der Wasserverbrauch ist zudem erheblich und die Verunreinigung der Gewässer mit bitterstoffhaltigem Waschwasser bedenklich. Es sind auch Entbitterungsverfahren bekannt, bei denen andere Lösungsmittel als Wasser zur Anwendung gelangen. Aber auch diese Verfahren sind mit erheblichen Nachteilen behaftet.

Aus der DE-C-350 100 ist ein Entbitterungsverfahren bekannt, bei welchem eine erste Menge Lupinen mit Wasser vermahlen wird, wobei ein Brei entsteht, von dem eine Extraktlösung durch Filtration abgetrennt wird. Mit dieser Extraktlösung wird eine zweite Menge Lupinen vermahlt, wobei sich die Extraktlösung weiter anreichert. Die weiter angereicherte Lösung wird wieder durch Filtration von dem bei der Naßmahlung entstandenen Brei abgetrennt. Dieser Vorgang wird mehrmals mit immer neuen Mengen Lupinen wiederholt, bis eine Extraktlösung erhalten wird, deren Aufnahmefähigkeit für Zucker- und Bitterstoffe erschöpft ist. Die erste Menge Lupinen wird dann noch einmal mit frischem Wasser ausgewaschen, wobei eine schwache Lösung entsteht, mit der die bei der 2., 3., 4., 5. usw. Naßmahlung erhaltenen festen Rückstände nachbehandelt werden, so daß durch wiederholte Anwendung der gleichen wäßrigen Zucker- und Bitterstofflösung die höchste Aufnahmefähigkeit für die löslichen Bestandteile erzielt wird. Die hochkonzentrierte Zuckerlösung, die man zum Schluß erhält, wird von Bitterstoffen und Alkaloiden dadurch befreit, daß sie mit Ammoniak schwach alkalisch gemacht wird und die freien Alkaloide mit einem Lösungsmittel extrahiert werden. Durch die Naßmahlung werden die Lupinen sehr stark zerkleinert.

Weiterhin ist aus der DE-C-531 828 ein Entbitterungsverfahren bekannt, bei welchem die Lupinen nach erfolgter Quellung einer stufenweisen Extraktion unterworfen werden. Die stufenweise Extraktion erfolgt in der Weise, daß während der ersten Extraktionsstufen mehrmals Extraktionsflüssigkeit durch die gequollenen Körner geleitet wird und daß bei den späteren Extraktionsstufen dieselbe Extraktionsflüssigkeit hintereinander über eine Mehrzahl von Portionen von Lupinenkörnern geleitet wird, so daß die gleiche Flüssigkeit wiederholt zur Extraktion von in verschiedenen Extraktionsstadien befindlichen Körnern benutzt wird und sich dabei an Alkaloiden und sonstigen Extraktstoffen anreichert. Danach erfolgt die Befreiung der Extraktionsflüssigkeit vom Alkaloid.

Neben der Verwendung der Bitterlupine als Nahrungs- und Futtermittel ist auch ihre Verwendung in gemahlener Form als pflanzenwachstumsförderndes Mittel bekannt. Die Wirkungen der gemahlenen Lupinen auf das Pflanzenwachstum sind jedoch bescheiden.

Es ist auch bekannt, daß der Extrakt aus Wurzeln einer Lupinenart (Lupinus albus) in Gegenwart von Gibberellinsäure sich stimulierend auf das Wachstum von Bohnen (Phaseolus multiflorus) auswirken kann. (Chemical Abstracts, Bd. 76, 13. März 1972, Nr. 11, Zsf. Nr. 56723z).

Die Aufgabe der Erfindung besteht darin, das gattungsgemäße Entbitterungsverfahren so auszugestalten, daß unter geringem Energieaufwand die Gewinnung eines entbitterten eiweißhaltigen Produktes und gleichzeitig eines wertvollen hochkonzentrierten bitterstoffhaltigen Extraktes aus den Samen von Bitterlupinen auf industrieller Basis ermöglicht wird.

Diese Aufgabe wird dadurch gelöst, daß die Lupinensamen zu Teilchen mit einer im Bereich zwischen 1 und 50 μm liegenden Korngröße vermahlen werden, daß in jedem Arbeitsgang zunächst die bitterstoffhaltigen Lupinenextraktlösungen in der Reihenfolge abnehmender Konzentration und dann das Lösungsmittel der Masse zugeführt, mit ihr vermischt und durch Filtration über ein Filtermedium von ihr wieder abgetrennt werden, auf dem die Mischung aus gemahlenen Lupinensamen und Extraktionsflüssigkeit ein Bett mit einer im Bereich zwischen 5 und 50 mm liegenden Schichthöhe bildet, und daß die jeweilige bitterstoffhaltige Lupinenextraktlösung höchster Konzentration nach ihrer weiteren Anreicherung bei einer Temperatur von unter 30 °C dehydratisiert wird.

Das erfindungsgemäße Verfahren hat den Vorteil, daß nur mit Wasser als Lösungsmittel und ohne Wärmezufuhr eine Bitterstoffextraktion vorgenommen wird, die zum einen keine qualitative Einbuße des Eiweißgehaltes der Lupine zur Folge hat und zum anderen den quantitativen Nährwertverlust in engen Grenzen hält, der sich ohnehin nur auf die sogenannten stickstofffreien Extraktionsstoffe beschränkt.

Da durch das erfindungsgemäße Verfahren die in den Lupinen enthaltenen Alkaloide mit ihren Begleitstoffen gleichzeitig in konzentrierter Form gewonnen werden und einer Verwendung zugeführt werden können, kann die geringe Nährwerteinbuße in wirtschaftlicher Hinsicht leicht kompensiert werden. Sie kann sogar mehr als kompensiert werden, da, wie sich in Versuchen gezeigt hat, die Konzentrate der Extrakte in flüssiger oder trockener Form ein hochwirksames, pflanzenwachstumförderndes Mittel sind, sowie als Insektizid und Herbizid und für pharmakologische Zwecke verwendet werden können.

Die Vorrichtung zur Durchführung des erfindungsgemäßen Verfahrens ist gekennzeichnet durch eine Mahleinrichtung, mindestens eine Extraktionsvorrichtung mit einem Filterboden zur Aufnahme der zu entbitternden Lupinen und eine Vielzahl von untereinander und mit der oder den Extraktionsvorrichtungen verschalteten Speicherräumen zur Aufnahme der Lupinenextraktlösungen.

Diese Vorrichtung hat den Vorteil, daß sie sich für den industriellen Einsatz eignet und mit ihr im Dauerbetrieb große Mengen entbitterter Lupinen für Nahrungs- und Futtermittelzwecke und große Mengen des sehr wertvollen, auf vielen Gebieten, insbesondere in der Land- und Forstwirtschaft, anwendbaren bitterstoffhaltigen Lupinenextraktes gewonnen werden können.

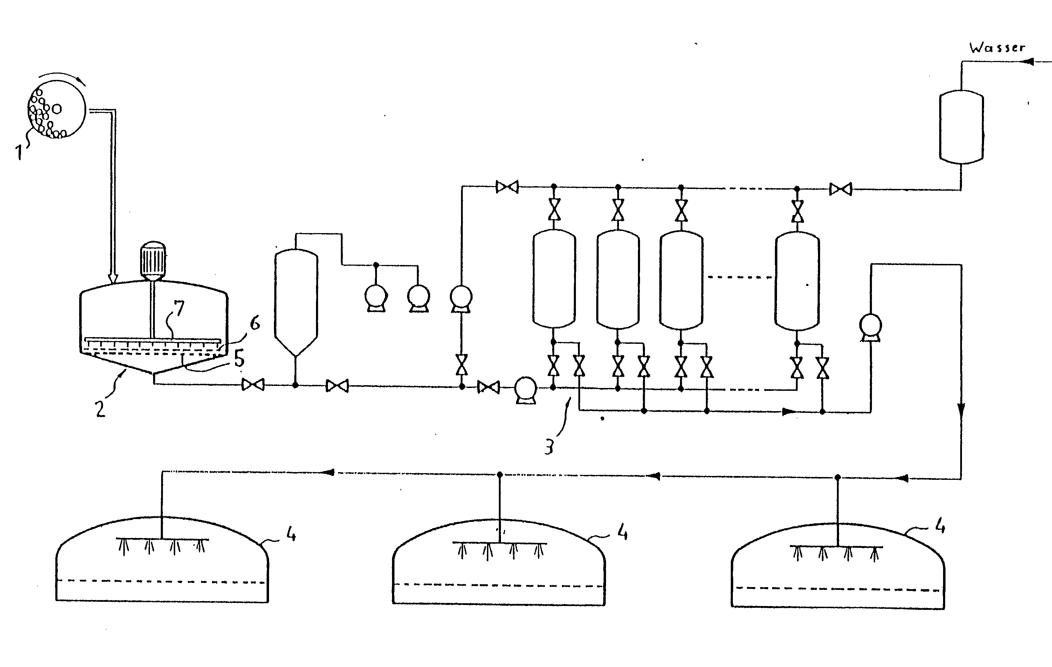

Weitere Merkmale und Vorteile der Erfindung ergeben sich aus der folgenden detaillierten Beschreibung des erfindungsgemäßen Verfahrens und der dazu verwendeten Vorrichtung, die, ergänzt durch eine Trocknungsvorrichtung zur Trocknung des gewonnenen Lupinenextraktes, in der Zeichnung in Form eines Schaltbildes näher dargestellt ist.

Die Vorrichtung zur Durchführung des erfindungsgemäßen Verfahrens, wie sie in der Zeichnung dargestellt ist, besteht im wesentlichen aus einer Kugelmühle 1, einem Flachbettfilterextraktor 2 und mehreren durch Leitungen untereinander und mit dem Extraktor 2 verschalteten Speicherbehältern 3. Neben diesen wesentlichen Elementen weist die Vorrichtung dem Verfahrensfachmann geläufige Bauteile, wie Ventile, Pumpen, Abscheider und Speicher auf, die in der Zeichnung zum Teil dargestellt, aber nicht näher bezeichnet sind. Ferner ist die Vorrichtung zur Durchführung des Verfahrens in der dargestellten Form mit einer mehrere Trocknungsgehäuse 4 aufweisenden Trocknungsvorrichtung verbunden, die zur Trocknung des durch die Auswaschung der Lupinen gewonnenen Alkaloid- und -begleitstoffextraktes dient. Der Extraktor 2 weist einen Filterboden auf, der aus einem Rost 5 und einem darauf aufliegenden feinstmaschigen Filtergewebe 6 besteht. Das Filtergewebe ist ein monofiles Gewebe aus Kunststoff, wie z. B. Polypropylen, Polyester oder Polyamid. Auf dem Filterboden des Extraktors wird die Trennung der Extraklösungen von der feinstgemahlenen Feststoffmasse durch Unterdruck vorgenommen.

Der Extraktor 2 ist ferner mit einem Rührwerk 7 ausgestattet, das Teil einer heb- und senkbaren Apparatur ist, die auch eine Vorrichtung zum Zustreichen des auf dem Filterboden sich bildenden Filterkuchens aufweist, zum Abheben des Filterkuchens von dem Filterboden dient und zum Reinigen des Filtergewebes verwendet werden kann.

Anstelle einer Kugelmühle könnte auch eine andere Mahleinrichtung verwendet werden, jedoch wird die Kugelmühle aufgrund ihrer hervorragenden Mahleigenschaften bevorzugt. Des weiteren können anstelle nur eines Extraktors mehrere durch Leitungen miteinander verbundene Extraktoren verwendet werden und kann auf die Behälterbatterie unter Umständen verzichtet werden, wenn die Extraktlösungen in den Extraktoren selbst und in den Leitungen, die sie verbinden, gespeichert werden können. Die Dauer der Speicherung der einzelnen Lösungsmengen wird verkürzt, je mehr Extraktoren eingesetzt werden.

Das erfindungsgemäße Verfahren läuft wie folgt ab. Eine Menge auszuwaschender, ungeschälter Lupinensamenkörner, auch Bohnen genannt, wird im Verhältnis 1 : 4 bis 1 : 10, vorzugsweise jedoch 1 : 5 bis 1 : 6, mit Wasser oder einer bei einer vorausgegangenen Verfahrensstufe gewonnenen Lupinenextraktlösung aus Wasser und bitterstoffhaltigen Extraktivstoffen angesetzt und in die Kugelmühle 1 gegeben, in welcher sie 2 bis 12 Stunden oder auch kürzer gemahlen werden. Die Mahlung bewirkt, daß die gegebenenfalls bereits zuvor grob zerkleinerten Lupinenbohnen nunmehr staubfein gemahlen werden, so daß sie eine Korngröße aufweisen, die im Bereich von 1 μ bis 50 μ liegt. Durch die Feinstmahlung der Lupinenbohnen findet ein Aufschluß der Zellulose statt, was für die Erzielung eines hohen Nährwerts der entbitterten Lupinen von großer Bedeutung ist.

Nach der Mahlung der Lupinenbohnen folgt die Auswaschung bzw. Extraktion im Extraktor 2. Die gemahlenen Lupinenbohnen, die nach der Naßmahlung in Form einer Schlämme vorliegen, werden auf dem Filterboden im Extraktor 2 in einer Schichthöhe von 5 bis 50 mm, vorzugsweise jedoch 10 bis 25 mm, aufgebracht und sodann nach dem Gegenstromprinzip stufenweise mit zuvor hergestellten Extraktlösungsmengen aus Wasser und bitterstoffhaltigen Extraktivstoffen der Lupinen und zuletzt mit reinem Wasser, das auch destilliertes Wasser sein kann, ausgewaschen.

Im einzelnen verläuft der Auswasch- bzw. Extraktionsprozess wie folgt. Die in den Speicherbehältern 3 gespeicherten Lupinenextraktlösungen werden in der Reihenfolge abnehmender Konzentration nacheinander mit der im Extraktor 2 befindlichen Extraktionsmasse aus gemahlenen Lupinenbohnen und Lupinenextraktlösung vermischt und von ihr wieder abgezogen, wobei angefangen wird mit der Extraktlösung höchster Konzentration und geendet wird mit der Extraktlösung geringster Konzentration. Zum Schluß wird reines Lösungsmittel, im Beispiel Wasser, mit der weitgehend ausgewaschenen Extraktionsmasse vermischt und als dünne Lösung davon wieder abgezogen. Bei diesem Verfahren erhöht sich die Konzentration jeder Lösung auf einen Wert, den die in der vorausgegangenen Stufe zugeführte Lösung vor ihrer Zumischung zur Extraktionsmasse hatte. Die jeweils im Extraktor weiter angereicherten Lösungen werden nach ihrer Durchführung durch den Extraktor jeweils in den Speicherbehälter zurückgeführt, in welchem zuvor eine Lösung mit der entsprechenden Konzentration gespeichert war. Die nach erfolgter Auswaschung einer Menge gemahlener. Lupinenbohnen vorliegende Lösung höchster Konzentration wird zur Trocknung in den Trocknungsgehäusen 4 abgeführt und durch frisches Lösungsmittel ersetzt. Die ausgewaschene Extraktionsmasse wird aus dem Extraktor 2 entfernt und durch frisch gemahlene Lupinenbohnen ersetzt. Danach folgt ein neuer Auswasch- bzw. Extraktionsvorgang in entsprechender Weise. Der gesamte Extraktionsprozess wird ohne Wärmezufuhr durchgeführt.

Zur Herstellung der Lupinenextraktlösungen unterschiedlicher Konzentration wird zu Beginn des Verfahrens einer frisch eingebrachten Charge von feinstgemahlenen Lupinenbohnen Wasser zugemischt, so daß eine Lösung entsteht, die von der Extraktionsmasse wieder abgezogen wird, wobei die Konzentration dieser Lösung kontinuierlich abnimmt. Die immer dünner werdende Lösung wird in mehrere Mengen unterschiedlicher Konzentration aufgeteilt und in den Behältern 3 gespeichert. Dann wird die ausgelaugte Extraktionsmasse durch eine frische Charge feinstgemahlener Lupinenbohnen ersetzt, und es werden die Lösungsmengen nacheinander dem aus den feinstgemahlenen Lupinenbohnen bestehenden Extraktionsgut jeweils zugemischt und davon wieder abgeführt, wobei sich die Konzentration jeder Lösungsmenge auf den Wert erhöht, den die in der vorhergehenden Stufe zugeführte Lösungsmenge vor ihrer Zumischung zu dem Extraktionsgut hatte. Auf diese Weise werden die Konzentrationswerte der einzelnen Extraktlösungsmengen in den Behältern 3 schrittweise erhöht, bis sich ein gewünschtes Konzentrationsgefälle bei den Lösungen ausgebildet hat.

Ist das Konzentrationsgefälle der Lösungen einmal eingestellt, kann das Verfahren kontinuierlich durchgeführt werden, wobei nach jedem Zyklus nur die extrahierten Lupinenbohnen jeweils durch frische ersetzt werden müssen und zu berücksichtigen ist, daß zum Schluß jeden Zykluses die Auswaschung der Lupinen mit reinem Wasser bzw. destilliertem Wasser, zu erfolgen hat, da ja mit dem erfindungsgemäßen Verfahren zweierlei erreicht werden soll : einmal die möglichst vollständige Entbitterung der Lupinen und zum anderen die Konzentration und Gewinnung der in den Lupinen enthaltenen Bitterstoffen und Begleitstoffen.

Für die erfolgreiche Durchführung der Auswaschung bzw. Extraktion der Lupinenbohnen ist die Einhaltung bestimmter Schichthöhen der Extraktionsmasse bzw. des durch Absaugen der Extraktlösungen entstehenden Filterkuchens im Extraktor von Bedeutung. Nachdem zu Beginn jeden Auswaschvorgangs die Extraktionsmasse auf dem Filterboden des Extraktors eine Schichthöhe von 5 bis 50 mm, vorzugsweise jedoch 10 bis 25 mm haben soll, sollte der in der Extraktionsvorrichtung am Ende jeden Auswaschvorgangs entstehende Filterkuchen im noch feuchten Zustand eine Schichthöhe von nur wenigen Millimetern bis maximal 30 mm haben. Vorzugsweise sollte die Schichthöhe des feuchten Filterkuchens 5 bis 15 mm betragen. Eine solche Schichthöhe des feuchten Filterkuchens entspricht einer Schichthöhe im trockenen Zustand des Filterkuchens von 2,5 bis 6 mm, und diese Schichthöhe entspricht einem Gewicht von 2 bis 5 kg/m$^2$.

An den Auswaschvorgang schließt sich ein Trocknungsvorgang zur Trocknung des Filterkuchens an, um die im Filterkuchen enthaltene Restfeuchtigkeit zu entziehen. Die Trocknung des Filterkuchens kann durch Ausdampfen der Feuchtigkeit oder durch Auspressen erfolgen. In jedem Fall ist es von Vorteil, wenn die in dem Filterkuchen nach dem Auswaschen enthaltene Restfeuchtigkeit möglichst gering ist. Zu diesem Zweck wird der zum Absaugen der Extraktionsflüssigkeit angelegte Unterdruck stufenweise gesteigert. Durch kontinuierliches Zustreichen von sich in dem Filterkuchen bildenden Rissen während des Absaugens wird auf diese Weise am Ende des Extraktionsvorganges ein Filterkuchen mit einer Restfeuchigkeit von nur 40 bis 50 % erhalten. Dies bedeutet, daß nur ungefähr die gleiche Menge an Wasser dem Filterkuchen bei der Trocknung entzogen werden muß, die die Trockensubstanzmenge des Filterkuchens ausmacht.

Die Trocknung des Filterkuchens kann im Extraktor selbst vorgenommen werden, indem künstlich Wärme zugeführt wird oder der Filterkuchen der Sonneneinstrahlung ausgesetzt wird. Der Filterkuchen kann aber auch außerhalb des Extraktors durch künstliche oder natürliche Wärmezufuhr oder, wie bereits erwähnt, durch Auspressen der Restfeuchtigkeit getrocknet werden. Das auf diese Weise erhaltene Trockenprodukt aus entbitterten Lupinen stellt ein hochwertiges Nahrungs- und Futtermittel dar, dem die Bitterstoffe bis auf einen Restgehalt von 0,002 % entzogen sind und dessen Eiweiß- und Fettgehalt nahezu dem der Lupinen vor der Entbitterung entspricht.

Der die Bitterstoffe enthaltende Lupinenextrakt, der einen Trockensubstanzgehalt von 10 bis 30 %, vorzugsweise jedoch 20 bis 25 %, hat und neben den wasserlöslichen Alkaloiden auch Kohlenhydrate, Fette, Eiweißstoffe und Mineralsalze in geringen Mengen enthält, wird, wie bereits erwähnt, der Trocknung zugeführt, der eine Aufkonzentrierung vorgeschaltet sein kann. Die Trocknung des Extraktes erfolgt dadurch, daß er in einem Strom entfeuchteter Luft oder in einem Inertgasstrom geringer Temperatur, d. h. unter 30 °C, auf einen gasdurchlässigen Träger in den Trocknungsgehäusen 4 der Trocknungsvorrichtung aufgesprüht wird.

Das auf diese Weise erhaltene Trockenprodukt stellt ein hochwertiges Mehrzweckmittel, insbesondere für die Land- und Forstwirtschaft, dar. Angewendet in Form einer dünnen, wässrigen Lösung mit einer 0,2 bis 5 %-igen Trockensubstanzkonzentration oder in Pulverform bewirkt es bei den Pflanzen eine erhebliche Wachstumsbeschleunigung und Wuchsvergrößerung. Je nach Pflanzenart können die Ernteerträge durch Anwendung des Lupinenextraktes um 5 bis 30 % gesteigert werden. Bei geeigneter Dosierung, die durch Versuche leicht ermittelbar ist, wirkt der bitterstoffhaltige Lupinenextrakt auch gegen viele Pflanzenschädlinge.

Das erfindungsgemäße Entbitterungsverfahren kann unabhängig von einem vor- oder nachzuschaltenden Verfahren zur Gewinnung der Öle aus den Lupinen durchgeführt werden.

Die Vorschaltung nach der Erfindung ist jedoch von Vorteil, da der bei der Extraktion entstehende Filterkuchen in ein Granulat zerkleinert werden kann und die Granulatform die nachfolgende Ölgewinnung erheblich vereinfacht.

## Patentansprüche

1. Verfahren zur Entbitterung der Samen von Bitterlupinen, bei dem in aufeinanderfolgenden Arbeitsgängen jeweils eine frische Menge naßgemahlener Lupinensamen einer stufenweisen Extraktion durch aufeinanderfolgende Einwirkung von mehreren, im vorausgegangenen Arbeitsgang angereicherten bitterstoffhaltigen Lupinenextraktlösungen unterschiedlich starker Konzentration und Lösungsmittel als Extraktionsflüssigkeit unterworfen wird, wobei als Lösungsmittel Wasser verwendet wird und die Extraktion im wesentlichen ohne Wärmezufuhr durchgeführt wird, dadurch gekennzeichnet, daß die Lupinensamen zu Teilchen mit einer im Bereich zwischen 1 und 50 μm liegenden Korngröße vermahlen werden, daß in jedem Arbeitsgang zunächst die bitterstoffhaltigen Lupinenextraktlösungen in der Reihenfolge abnehmender Konzentration und dann das Lösungsmittel der Masse zugeführt, mit ihr vermischt und durch Filtration über ein Filtermedium von ihr wieder abgetrennt werden, auf dem die Mischung aus gemahlenen Lupinensamen und Extraktionsflüssigkeit ein Bett mit einer im Bereich zwischen 5 und 50 mm liegenden Schichthöhe bildet, und daß die jeweilige bitterstoffhaltige Lupinenextraktlösung höchster Konzentration nach ihrer weiteren Anreicherung bei einer Temperatur von unter 30 °C dehydratisiert wird.

2. Verfahren nach Anspruch 1, dadurch gekennzeichnet, daß die Naßmahlung der Lupinensamen unter Zusatz von einer bitterstoffhaltigen Lupinenextraktlösung höchster Konzentration durchgeführt wird.

3. Verfahren nach Anspruch 1 oder 2, dadurch gekennzeichnet, daß die Naßmahlung der Lupinensamen in einer Kugelmühle erfolgt.

4. Verfahren nach einem der vorhergehenden Ansprüche, dadurch gekennzeichnet, daß die Lupinensamen mit Extraktionsflüssigkeit im Verhältnis von 1 : 4 bis 1 : 10 vermischt werden.

5. Verfahren nach einem der vorhergehenden Ansprüche, dadurch gekennzeichnet, daß die jeweilige bitterstoffhaltige Lupinenextraktlösung mittels Unterdruck durch das Filtermedium hindurch von der Mischung abgesaugt wird.

6. Verfahren nach einem der vorhergehenden Ansprüche, dadurch gekennzeichnet, daß die Mischung aus gemahlenen Lupinensamen und Extraktionsflüssigkeit vor der Abtrennung der jeweiligen Extraktlösung gerührt wird.

7. Verfahren nach einem der vorhergehenden Ansprüche, dadurch gekennzeichnet, daß die Dehydratation der Extraktlösungen in einem entfeuchteten Luft- oder Inertgasstrom erfolgt.

8. Bitterstoffhaltiger Lupinensamen-Trockenextrakt, erhältlich durch das Verfahren gemäß einem der Ansprüche 1 bis 7.

9. Verwendung des bitterstoffhaltigen Lupinensamen-Trockenextraktes nach Anspruch 8, in Pulverform oder in Wasser gelöst, als pflanzenwachstumsförderndes Mittel.

## Claims

1. Method for debittering the seeds of bitter lupines in which in successive working operations in each case a fresh amount of wet-ground lupine seeds is subjected to a stepwise extraction by successive action of a plurality of bitter-substance-containing lupine extract solutions concentrated in the preceding working operation and of differently strong concentration and solvent as extraction liquid, water being used as solvent and the extraction being carried out substantially without heat supply, characterized in that the lupine seeds are ground to particles with a grain size lying in the range between 1 and 50 μm, that in each working operation firstly the bitter-substance-containing lupine extract solutions in the order of decreasing concentration and then the solvent are supplied to the mass, mixed therewith and by filtration again separated therefrom via a filter medium on which the mixture of ground lupine seeds and extraction liquid forms a bed with a layer height lying in the range between 5 and 50 mm, and that the particular bitter-substance-containing lupine extract solution of highest concentration after further concentration is dehydrated at a temperature below 30 °C.

2. Method according to claim 1, characterized in that the wet grinding of the lupine seeds is carried out with addition of a bitter-substance-containing lupine extract solution of highest concentration.

3. Method according to claim 1 or 2, characterized in that the wet grinding of the lupine seeds is carried out in a ball mill.

4. Method according to any one of the preceding claims, characterized in that the lupine seeds are mixed with extraction liquid in the ratio of 1 : 4 to 1 : 10.

5. Method according to any one of the preceding claims, characterized in that the respective bitter-substance-containing lupine extract solution is extracted from the mixture through the filter medium by means of reduced pressure.

6. Method according to any one of the preceding claims, characterized in that the mixture of ground lupine seeds and extraction liquid is stirred prior to the separation of the particular extract solution.

7. Method according to any one of the preceding claims, characterized in that the dehydration of the extract solutions takes place in a demoisturized air or inert gas stream.

8. Bitter-substance-containing lupine seed dry extract, obtainable by the method according to any one of claims 1 to 7.

9. Use of the bitter-substance-containing lupine seed dry extract according to claim 8 in powder form or dissolved in water as agent promoting plant growth.

**Revendications**

1. Procédé pour enlever leur amertume aux graines de lupins amers, dans lequel, en des opérations successives, une quantité fraîche de graines de lupins moulues par voie humide est soumise chaque fois à une extraction par étapes, par action successive de plusieurs solutions d'extrait de lupins enrichies dans l'opération précédente et contenant le principe amer, les solutions étant de concentrations différentes, et à un solvant constituant un liquide d'extraction et constitué par de l'eau, l'extraction se faisant essentiellement sans apport de chaleur, caractérisé en ce que les graines de lupins sont moulues en particules ayant un calibre compris entre 1 et 50 microns ; que, dans chaque opération, d'abord les solutions d'extrait de lupins contenant le principe amer sont adjointes à la masse par ordre de concentration décroissante, qu'ensuite le solvant y est adjoint ; que solutions et solvant sont mélangés avec la masse et qu'ils en sont de nouveau séparés par filtration par l'intermédiaire d'un milieu filtrant, sur lequel le mélange composé des graines de lupins moulues et du liquide d'extraction forme un lit ayant une hauteur de couche comprise entre 5 et 50 mm et que la solution d'extrait de lupins correspondante, qui contient le principe amer et qui est de la plus haute concentration, est déshydratée, après son enrichissement suivant à une température inférieure à 30 °C.

2. Procédé suivant la revendication 1, caractérisé en ce que la mouture par voie humide des graines de lupins est exécutée avec une addition d'une solution d'extrait de lupins qui contient le principe amer et qui est de la plus haute concentration.

3. Procédé suivant la revendication 1 ou 2, caractérisé en ce que la mouture des graines de lupin par voie humide s'exécute dans un broyeur à boulets.

4. Procédé suivant une des revendications précédentes, caractérisé en ce que les graines de lupins sont mélangées avec le liquide d'extraction dans un rapport compris entre 1/4 et 1/10.

5. Procédé suivant une des revendications précédentes, caractérisé en ce que chaque solution d'extrait de lupins qui contient le principe amer est aspirée du mélange par dépression, à travers le milieu filtrant.

6. Procédé suivant une des revendications précédentes, caractérisé en ce que le mélange composé de graines de lupins moulues et de liquide d'extraction est mélangé avant la séparation de chaque solution d'extrait.

7. Procédé suivant une des revendications précédentes, caractérisé en ce que la déshydratation des solutions d'extrait se réalise dans un courant déshydraté d'air ou de gaz inerte.

8. Extrait sec de graines de lupins contenant un principe amer, pouvant être obtenu par le procédé conforme à une des revendications 1 à 7.

9. Utilisation de l'extrait sec de graines de lupins contenant un principe amer et conforme à la revendication 8, sous forme de poudre ou dissous dans l'eau, comme produit stimulant la croissance des plantes.